(19) Europäisches Patentamt
European Patent Office
Office européen des brevets

(11) Veröffentlichungsnummer: **0 017 122**
**B1**

(12) # EUROPÄISCHE PATENTSCHRIFT

(45) Veröffentlichungstag der Patentschrift: **15.06.83**

(51) Int. Cl.$^3$: **A 61 K 7/06**

(21) Anmeldenummer: **80101536.3**

(22) Anmeldetag: **24.03.80**

(54) **Verwendung neuer quartärer Polysiloxanderivate in Haarkosmetika, sowie diese enthaltende Haarwasch- und Haarbehandlungsmittel.**

(30) Priorität: **29.03.79 DE 2912484**

(43) Veröffentlichungstag der Anmeldung: **15.10.80 Patentblatt 80/21**

(45) Bekanntmachung des Hinweises auf die Patenterteilung: **15.06.83 Patentblatt 83/24**

(84) Benannte Vertragsstaaten: **AT BE CH DE FR GB IT NL SE**

(56) Entgegenhaltungen:
**US-A-3 389 160**
**US-A-4 005 117**
**US-A-4 006 176**
**US-A-4 185 087**

(73) Patentinhaber: **Henkel Kommanditgesellschaft auf Aktien, Postfach 1100 Henkelstrasse 67, D-4000 Düsseldorf 1 (DE)**

(72) Erfinder: **Petzold, Manfred, Am Falder 93, D-4000 Düsseldorf 13 (DE)**
Erfinder: **Uphues, Günther, Himmelgeister Strasse 143, D-4000 Düsseldorf (DE)**
Erfinder: **Hempel, Hans Ulrich, Dr., Wiesengrund 5, D-5063 Overath/Vilkerath (DE)**
Erfinder: **Scheuermann, Fanny, geb. Esano-Solomon, Dr., Volmerswertherstrasse 66, D-4000 Düsseldorf (DE)**

**EP 0 017 122 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verwendung neuer quartärer Polysiloxanderivate in Haarkosmetika, sowie diese enthaltende Haarwasch- und Haarbehandlungsmittel

Die Erfindung betrifft die Verwendung neuer quartärer Polysiloxan-Ammonium-Derivate in Haarkosmetika zur Verbesserung der Kämmbarkeit, Weichheit und Fülle, sowie diese enthaltende Haarwasch- und Haarbehandlungsmittel.

Es wurde gefunden, daß quartäre Polysiloxan-Ammonium-Derivate der allgemeinen Formel

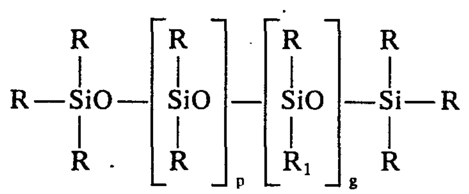

in der R einen Alkylrest mit 1—4 Kohlenstoffatomen oder einen Arylrest, p die Zahlen von 0—300, vorzugsweise 10—50 und g die Zahlen 1—75, vorzugsweise 1—12 und $R_1$ den Rest

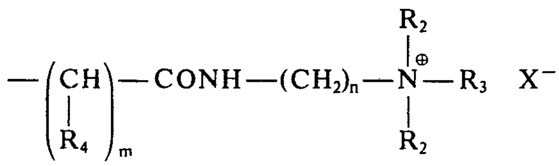

darstellt, wobei $R_2$ einen Alkyl- oder Hydroxyalkylrest mit 1—3 Kohlenstoffatomen, $R_3$ einen Rest gleich $R_2$ oder Aryl-$CH_2$- oder den Allylrest, $R_4$ Wasserstoff oder den Methylrest, $X^-$ die Anionen $Cl^-$, $Br^-$, $J^-$, $CH_3SO_4^-$ oder $C_2H_5-SO_4^-$, und m die Zahlen 2—10, vorzugsweise 10, n die Zahlen 2—4, vorzugsweise 3 bedeuten in vorteilhafter Weise für die Herstellung von Haarkosmetika geeignet sind. Haarwasch- und Haarbehandlungsmittel, die diese quartären Polysiloxan-Ammonium-Derivate enthalten, verleihen den damit behandelten Haaren eine ausgezeichnete Kämmbarkeit, sowie Weichheit und Fülle.

Die Herstellung der erfindungsgemäß zu verwendenden Polysiloxan-Ammonium-Derivate erfolgt nach bekannten Verfahren der organischen Chemie. Gemäß einer Herstellungsweise werden Alkensäuremethylester nach bekannten Verfahren in Gegenwart eines Platinkatalysators an Alkyl(Aryl)-dichlorsilan addiert. Durch Cohydrolyse des Adduktes mit Dialkyl (Aryl)-dichlorsilan und Trialkyl(Aryl)-chlorsilan gelangt man zu den kammartig gebauten Polysiloxanen mit Alkylensäuremethylestergruppen in den Seitenketten. Diese werden mit Aminoalkylentertiäraminen umgesetzt und das erhaltene Amidotertiäramin wird anschließend mit üblichen Alkylierungsmitteln, wie zum Beispiel Benzylchlorid, Methylchlorid, Dimethylsulfat, gegebenenfalls in Gegenwart von Lösungsmitteln, quaterniert. Die Reaktion verläuft dabei nach folgendem Schema:

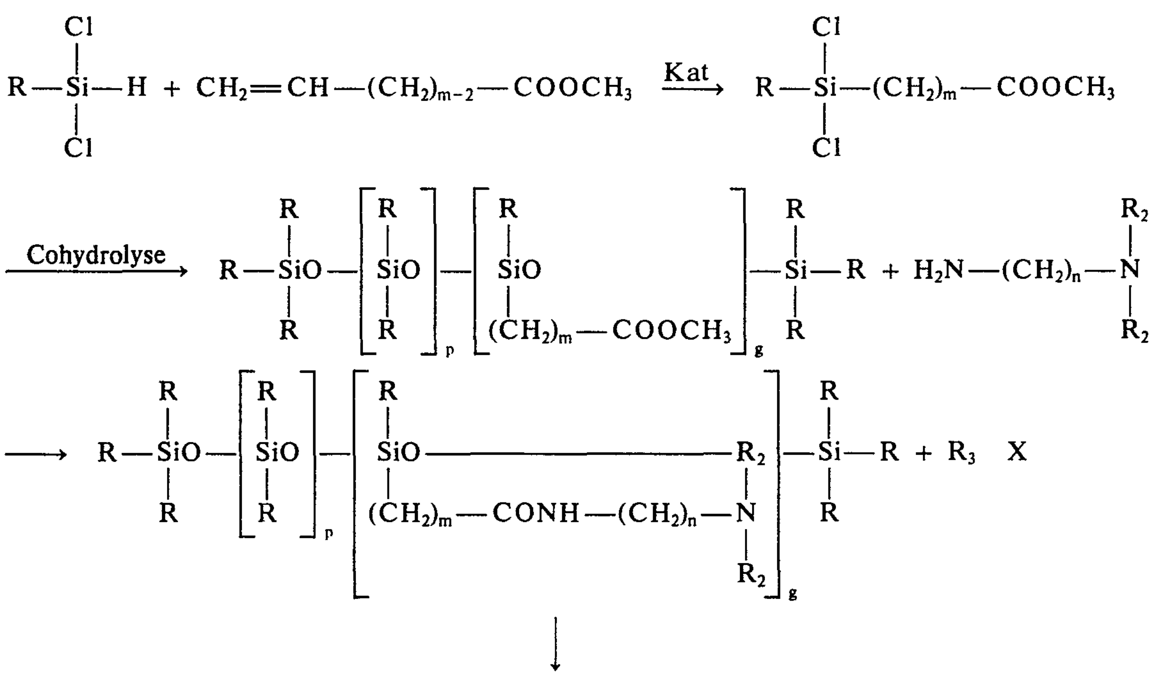

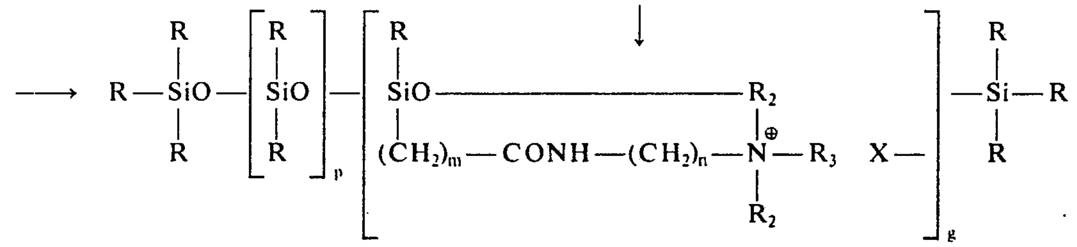

Als Reaktionskomponente für die Umsetzung der Alkyl(Aryl)-dichlorsilane zu den entsprechenden Carbonsäureestern können alle, eine endständige Doppelbindung tragenden aliphatischen Carbonsäuremethylester dienen, wie zum Beispiel Methylester der Acrylsäure, Methacrylsäure, Undecylensäure. Besondere Bedeutung kommt dabei, sowohl von der leichten Weiterverarbeitbarkeit als auch von der besonders guten kosmetischen Wirkung des Endproduktes her, dem Undecylensäuremethylester zu.

Als Reaktionskomponente für die Aminolyse der Polysiloxanalkylencarbonsäureester zu den entsprechenden Amidoaminen können N,N-Dialkylaminoalkylamine eingesetzt werden, deren Alkyl- bzw. Hydroxyalkylsubstituenten 1—3 Kohlenstoffatome besitzen und deren Kohlenstoffatomzahl in der Alkylkette 2—4, vorzugsweise 3 beträgt, wie z. B. Dimethylamino-ethylamin, -propylamin, -butylamin, Diethylamino-ethylamin, -propylamin, -butylamin, Dipropylamino-ethylamin, -propylamin, -butylamin, Di-2-hydroxyethylamino-ethylamin, -propylamin, -butylamin, Dibutylamino-propylamin. Unter diesen kommt dem Dimethylaminopropylamin und Diethylaminopropylamin besondere Bedeutung zu.

Für die Quaternierungsreaktion lassen sich als Alkylierungsmittel alle für diesen Zweck gebräuchlichen Verbindungen einsetzen, wie z. B. Methylchlorid, Allylchlorid, Benzylchlorid, Dimethylsulfat, Diethylsulfat, Methylbromid, Methyljodid, Benzylbromid. Für die Durchführbarkeit der Quaternierungsreaktion ist in fast allen Fällen die Mitverwendung von Alkohol als Lösungsmittel erforderlich, da bei nur wäßriger Verdünnung gelartige, nicht zu rührende Produkte anfallen. Als bevorzugtes Quaternierungsmittel ist Benzylchlorid anzusehen. Die erfindungsgemäß zu verwendenden Polysiloxan-Ammonium-Derivate selbst stellen flüssige oder feste Substanzen dar, die je nach Größe der Polysiloxankette im Wasser dispergierbar oder löslich sind. In vielen Fällen werden die Reaktionsprodukte als klare bis opaleszente, viskose, ca. 50%ige Lösungen erhalten.

Bevorzugt zu verwendende Verbindungen unter den Polysiloxan-Ammonium-Derivaten stellen demnach Produkte folgender allgemeiner Formel dar:

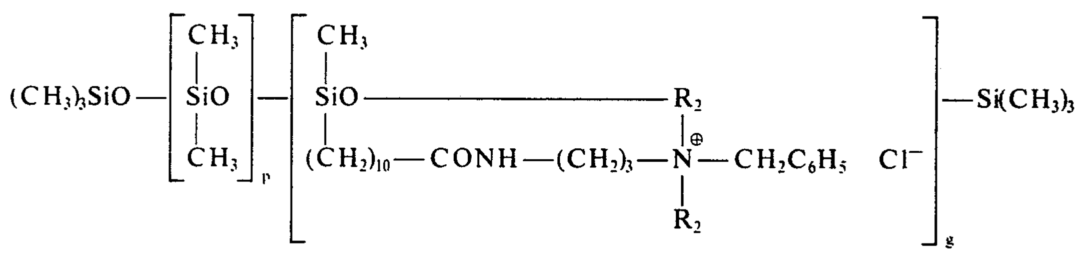

in denen $R_2$ den Methyl- oder Ethylrest, p die Zahlen 10—50 und g die Zahlen 1—12 bedeuten.

Eine Isolierung der Polysiloxan-Ammonium-Derivate in reiner Form ist für den Einsatz in den kosmetischen Präparationen nicht erforderlich, da die Einarbeitung in die Haarwasch- und Haarbehandlungsmittel in Gestalt der viskosen wäßrigen Lösung erfolgen kann. Aus praktischen Gründen sollte die Konzentration der wäßrigen Lösung nicht wesentlich unter 50 Gewichtsprozent liegen.

Die Einarbeitung der wäßrigen Lösungen der Polysiloxan-Ammonium-Derivate in die Haarwasch- und Haarbehandlungsmittel erfolgt in üblicher Weise durch einfaches Einrühren. Dabei bewegt sich die zugesetzte Menge in den Grenzen von 0,1 bis 10 Gewichtsprozent, vorzugsweise 0,5—5 Gewichtsprozent, berechnet als reines Polysiloxan-Ammonium-Derivat und bezogen auf das gesamte haarkosmetische Mittel.

In die erfindungsgemäßen Haarwasch- und Haarbehandlungsmittel können neben dem Polysiloxan-Ammonium-Derivat alle üblichen Zusatzstoffe, wie Proteinhydrolysate, Kräuterauszüge, Rückfettungsmittel, Schaumstabilisatoren, Antischuppenmittel, Parfümöle, Konservierungsmittel, haarfestigende Kunstharze und andere in den für den jeweiligen Verwendungszweck gebräuchlichen Mengen eingearbeitet werden. Aber auch bei dem Einsatz oberflächenaktiver Verbindungen bestehen uneingeschränkte Kombinationsmöglichkeiten, da die erfindungsgemäß einzusetzenden Polysiloxan-Ammonium-Derivate sowohl mit kationaktiven und nichtionogenen Tensiden als auch mit anionaktiven Tensiden sehr gut verträglich sind. Hierdurch wird die Möglichkeit geschaffen, einen sehr gut avivierenden Wirkstoff in Shampoos auf Basis anionaktiver Tenside, wie zum Beispiel Alkylsulfaten,

Alkyläthersulfaten einzuarbeiten. Bei diesem Einsatz in erfindungsgemäßen Haarwaschmitteln wird durch die Polysiloxan-Ammonium-Derivate weder die Viskosität noch das Schaumverhalten oder die Waschkraft des Shampoos beeinträchtigt.

Als Beispiele für haarkosmetische Zubereitungen, denen durch den Zusatz der Polysiloxan-Ammonium-Derivate vorteilhafte Eigenschaften verliehen werden können, sind Shampoos, Haarfestiger, Haarkuren, Haarregeneratoren. Haarsprays, Kurspülungen, Haarwässer zu nennen.

Die erfindungsgemäßen Haarwasch- und Haarbehandlungsmittel verleihen dem damit behandelten Haar im Hinblick auf die üblichen Pflegemaßnahmen außergewöhnlich günstige Eigenschaften. So wird die Naßkämmbarkeit der Haare wesentlich verbessert, das trockene Haar ist weich und angenehm im Griff und von guter Fülle und läßt sich ohne Schwierigkeiten frisieren. Die statische Aufladung des trockenen Haares ist weitgehend herabgesetzt und das behandelte Haar behält die frisiertechnisch günstigen Eigenschaften über einen längeren Zeitraum bei. Über die wesentliche Verbesserung der frisiertechnischen Eigenschaften des Haares hinaus, bewirkt die Behandlung mit den erfindungsgemäßen Haarwasch- und Haarbehandlungsmitteln eine erhebliche Steigerung des Glanzes der behandelten Haare. Weiterhin ist die besonders gute physiologische Verträglichkeit der einzusetzenden Produkte zu erwähnen.

Die nachfolgenden Beispiele sollen den Gegenstand der Erfindung näher erläutern, ohne ihn jedoch hierauf zu beschränken.

## Beispiele

Zunächst wird die Herstellung der Polysiloxan-Ammonium-Derivate beschrieben.

$A_1$) Herstellung der Methyl-dichlorsilan-Undecylensäureester-Addukte:

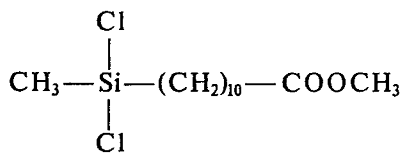

(1)

Die Herstellung erfolgte durch Addition von Undecylensäuremethylester an Methyldichlorsilan gemäß den Angaben von Nasser Saghian und David Gertner in J. Am. Oil Chemist's Soc. 51 (1974), Seite 363 ff.

$A_2$) Herstellung der Polysiloxane kammartiger Struktur mit Undecansäuremethylestergruppen in der Seitenkette:

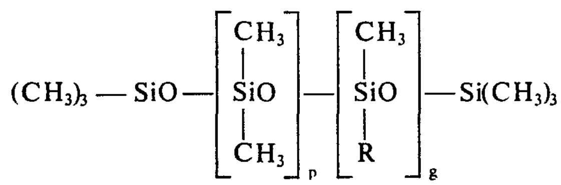

(2)

$R = -(CH_2)_{10}-COOCH_3$
$p = 31$
$g = 6$

Die Herstellung erfolgt durch Cohydrolyse der unter $A_1$ hergestellten Verbindung (1) mit Dimethyldichlorsilan und Trimethylchlorsilan auf folgendem Wege: 360 g (1,15 Mol) der Verbindung (1), 890 g (6,9 Mol) Dimethyldichlorsilan und 41,4 g (0,38 Mol) Trimethylchlorsilan wurden gemischt und bei Raumtemperatur (Kühlung mit Eis) in 7 Liter Wasser eingetropft. Nach beendeter Zugabe wurde 1 Stunde bei Raumtemperatur nachgerührt. Die Siloxanphase wurde abgetrennt, mit Natriumhydrogencarbonatlösung und Wasser neutral gewaschen, über Natriumsulfat getrocknet und filtriert. Das erhaltene Rohprodukt wurde mit 3% Bleicherde (Tonsil LFF 80) versetzt und unter Stickstoff 16 Stunden lang auf 120°C erhitzt. Danach wurde die Bleicherde abfiltriert und das Filtrat zur Entfernung leichtflüchtiger Anteile bis 180°C/18 mbar andestilliert. Es wurde ein klares, leicht gelb gefärbtes Öl mit einer Viskosität von 187 m Pas (20°C), einer Verseifungszahl von 83,3 und einem Molgewicht von 4040 erhalten.

In völlig analoger Weise wurden weitere Polysiloxane kammartiger Struktur mit Undecansäuremethylestergruppen in der Seitenkette hergestellt, die in nachstehender Tabelle 1 aufgeführt sind.

Tabelle 1

| Verbindung | p | g | VZ | Viskosität m Pas (20°C) | Mol. Gew. |
|---|---|---|---|---|---|
| $B_2$ | 16 | 3 | 83,3 | 90 | 2000 |
| $C_2$ | 44 | 3 | 40,4 | 33 | 4200 |
| $D_2$ | 30 | 1 | 21,2 | 71 | 2650 |
| $E_2$ | 31 | 5 | 74,7 | 106 | 3750 |

$A_3$) Herstellung der tertiären Aminopolysiloxane durch Aminolyse der Polysiloxane kammartiger Struktur mit Undecansäuremethylestergruppen in der Seitenkette.
Für die Aminolyse diente Dimethylaminopropylamin (DMAP) beziehungsweise Diethylaminopropylamin (DEAP).

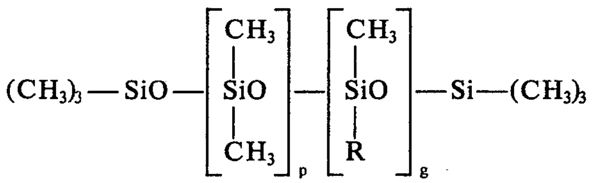

(3)

R = 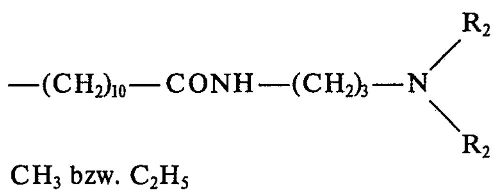

$R_2$ = $CH_3$ bzw. $C_2H_5$

Die Herstellung erfolgte durch Aminolyse der unter $A_2$ hergestellten Verbindung 2 (p=31, g=6) mit Diethylaminopropylamin auf folgendem Wege:
101 g (0,15 Äquivalent des Polysiloxanderivats $A_2$ und 39 g (0,3 Mol) 3-Diethylaminopropylamin wurden gemischt und das Gemisch wurde unter Einleiten eines schwachen Stickstoffatomes langsam erhitzt. Ab 139°C begann die Bildung von Destillat. Das Erhitzen wurde bis zu einer Höchsttemperatur von 170°C fortgesetzt und das Gemisch 90 Minuten bei dieser Temperatur gehalten. Während dieser Zeit sind 4,7 g Destillat angefallen. Nach Abkühlen auf 40°C wurde im Wasserstrahlvakuum bis zu einer Temperatur von 150°C das nicht umgesetzte 3-Diethylaminopropylamin entfernt. Es wurden 112,5 g einer viskosen Flüssigkeit erhalten, für die nähere Angaben in der Tabelle 2 enthalten sind, nebst Daten analog hergestellter weiterer tertiärer Aminopolysiloxane.

Tabelle 2

| Verbindung | Ausgangs-Polysiloxan | Amin-komponente | Mol. Gew. theor. | % N-Amin | Löslich in verdünnter Essigsäure |
|---|---|---|---|---|---|
| $A_3$ | $A_2$ | DEAP | 4600 | 1,79 | klar |
| $B_3$ | $B_2$ | DMAP | 2100 | 1,96 | klar |
| $C_3$ | $C_2$ | DMAP | 4200 | 0,85 | dispergierbar |
| $D_3$ | $D_2$ | DMAP | 2600 | 0,36 | gerade noch mischbar |
| $E_3$ | $E_2$ | DMAP | 4100 | 1,6 | trüb |
| $H_3$ | $A_2$ | DMAP | 4400 | 1,8 | klar |

$A_4$) Herstellung der quartären Polysiloxan-Ammonium-Derivate durch Umsetzung der tertiären Aminopolysiloxane mit Benzylchlorid beziehungsweise anderen Quaternierungsmitteln.

Die Herstellung erfolgte durch Umsetzung des unter $A_3$ genannten tertiären Aminopolysiloxans mit Benzylchlorid auf folgendem Wege.

80 g (0,102 Äquivalent) des tertiären Aminopolysiloxans $A_3$ wurden mit 46,4 g Isopropanol und 46,8 g Wasser versetzt und auf 80°C erwärmt. Bei dieser Temperatur wurden innerhalb von 20 Minuten 12,8 g (0,102 Mol) Benzylchlorid zugetropft. Die anfangs trübe Mischung ist danach klar geworden. Unter Aufrechterhalten der Temperatur wurde 2 Stunden nachgerührt und danach abgekühlt. Es wurden 185,8 g an quartären Polysiloxan-Ammonium-Derivat in Form einer klaren, viskosen Flüssigkeit erhalten. Nähere Angaben hierfür, sowie für die analog hergestellten anderen Verbindungen finden sich in der Tabelle 3.

Tabelle 3

| Verbindung | tert. Aminopolysiloxan Ausgangsprodukt | Quaternierungsmittel | Wasserlöslichkeit |
|---|---|---|---|
| $A_4$ | $A_3$ | Benzylchlorid | etwas trüb |
| $B_4$ | $B_3$ | Benzylchlorid | etwas trüb |
| $C_4$ | $C_3$ | Benzylchlorid | sehr trüb |
| $D_4$ | $D_3$ | Benzylchlorid | unlöslich |
| $E_4$ | $E_3$ | Benzylchlorid | trüb |
| $H_4$ | $H_3$ | Benzylchlorid | etwas trüb |
| $J_4$ | $E_3$ | Dimethylsulfat | trüb |

Nachstehend werden einige Rezepturen von erfindungsgemäßen Haarwasch- und Haarbehandlungsmitteln mit einem Gehalt an Polysiloxan-Ammonium-Derivaten aufgeführt.

Shampoo

| | |
|---|---|
| Natriumlauryläthersulfat mit 27/28% Waschaktivsubstanz | 30,0 Gewichtsteile |
| Natriumchlorid | 2,0 Gewichtsteile |
| Kokosfettsäurediäthanolamid | 2,0 Gewichtsteile |
| Polysiloxan-Ammonium-Verbindung $A_4$ | 5,0 Gewichtsteile (als 50%ige wäßrige Lösung) |
| Zinkpyridinthion | 1,0 Gewichtsteile |
| Proteinhydrolysat | 1,0 Gewichtsteile |
| Duftkomponente | 1,0 Gewichtsteile |
| Wasser | 58,0 Gewichtsteile |

Shampoo

| | |
|---|---|
| Natriumlauryläthersulfat mit 35—37% Waschaktivsubstanz | 30,0 Gewichtsteile |
| Natriumchlorid | 1,0 Gewichtsteile |
| Polysiloxan-Ammonium-Verbindung $B_4$ | 8,0 Gewichtsteile (als 50%ige wäßrige Lösung) |
| Duftkomponente | 1,0 Gewichtsteile |
| Wasser | 60,0 Gewichtsteile |

Shampoo für fettes Haar

| | |
|---|---|
| Magnesiumlauryläthersulfat 29—31% Waschaktivsubstanz | 30,0 Gewichtsteile |
| Kokosfettsäurediäthanolamid | 5,0 Gewichtsteile |
| Polysiloxan-Ammonium-Verbindung $H_4$ | 5,0 Gewichtsteile (als 50%ige wäßrige Lösung) |

| | |
|---|---|
| Glycerin-$C_{8-18}$-fettsäureester + 7 Mol Äthylenoxid | 3,0 Gewichtsteile |
| Proteinhydrolysat | 1,0 Gewichtsteile |
| p-Hydroxybenzoesäuremethylester | 0,2 Gewichtsteile |
| Duftkomponente | 1,0 Gewichtsteile |
| Wasser | 54,8 Gewichtsteile |

Haarkurspülung

| | |
|---|---|
| Cetylalkohol | 3,0 Gewichtsteile |
| Vaselinöl | 2,0 Gewichtsteile |
| Polysiloxan-Ammonium-Verbindung $E_4$ | 5,0 Gewichtsteile (als 50%ige wäßrige Lösung) |
| $C_{8-18}$-Alkyl-dimethyl-aceto-betain | 5,0 Gewichtsteile |
| Zitronensäure | 1,0 Gewichtsteile |
| Kräuterauszüge | 1,0 Gewichtsteile |
| Parfümöl | 1,0 Gewichtsteile |
| Wasser | 82,0 Gewichtsteile |

Haarregenerator

| | |
|---|---|
| Cetylstearylalkohol | 4,0 Gewichtsteile |
| Zitronensäure | 1,0 Gewichtsteile |
| Parfüm, wasserlöslich | 1,0 Gewichtsteile |
| Polysiloxan-Ammonium-Verbindung $J_4$ | 3,0 Gewichtsteile (als 50%ige wäßrige Lösung) |
| Wasser | 91,0 Gewichtsteile |

Schnellhaarkur

| | |
|---|---|
| Cetylalkohol | 6,0 Gewichtsteile |
| Polysiloxan-Ammonium-Verbindung $A_4$ | 10,0 Gewichtsteile (als 50%ige wäßrige Lösung) |
| Ölsäuredecylester $C_{8-18}$-Alkyl-dimethyl-aceto-betain | 5,0 Gewichtsteile |
| Zitronensäure | 1,0 Gewichtsteile |
| Kräuterauszüge | 1,0 Gewichtsteile |
| Parfümöl | 1,0 Gewichtsteile |
| Wasser | 73,0 Gewichtsteile |

Haarspray

| | |
|---|---|
| Mischpolymeres aus Polyvinylpyrrolidon/Polyvinylacetat 60 : 40 | 4,0 Gewichtsteile |
| Polysiloxan-Ammonium-Verbindung $B_4$ | 2,0 Gewichtsteile (als 50%ige wäßrige Lösung) |
| Duftkomponente | 2,0 Gewichtsteile |
| Äthanol 96%ig | 92,0 Gewichtsteile |

Abfüllung: 40% Haarlack obiger Rezeptur
60% Treibgas (Difluordichlormethan-Monofluortrichlormethan 50 : 50)

**Patentansprüche**

1. Verwendung von Polysiloxan-Ammonium-Derivaten der allgemeinen Formel I

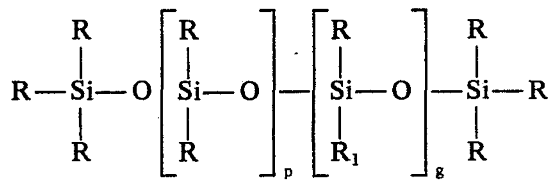

(I)

in der R einen Alkylrest mit 1–4 Kohlenstoffatomen oder einen Arylrest, p die Zahlen von 0–300, g die Zahlen 1–75 und $R_1$ den Rest

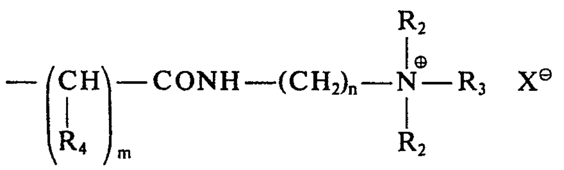

darstellt, wobei $R_2$ einen Alkyl- oder Hydroxyalkylrest mit 1—3 Kohlenstoffatomen, $R_3$ einen Rest gleich $R_2$ oder Aryl-$CH_2$- oder den Allylrest, $R_4$ Wasserstoff oder den Methylrest, $X^-$ die Anionen $Cl^-$, $Br^-$, $I^-$, $CH_3—SO_4^-$ oder $C_2H_5—SO_4^-$, m die Zahlen 2—10, und n die Zahlen 2—4 bedeuten, in Haarwasch- und Haarbehandlungsmitteln zur Verbesserung der frisiertechnischen Eigenschaften der Haare.

2. Verwendung von Polysiloxan-Ammonium-Derivaten nach Anspruch 1 der Formel II

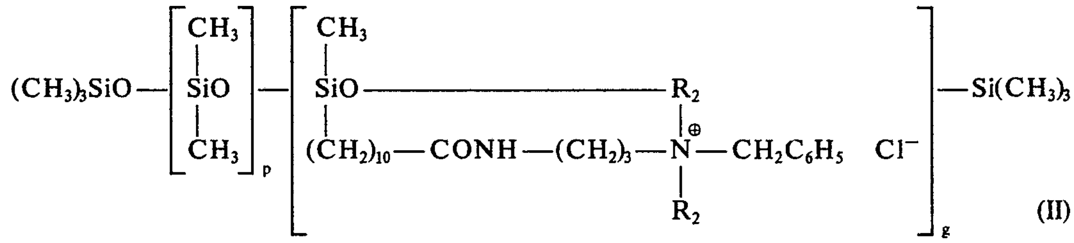

(II)

in der $R_2$ den Methyl- oder Ethylrest, p die Zahlen 10—50 und g die Zahlen 1—12 bedeuten.

3. Haarwasch- und Haarbehandlungsmittel, gekennzeichnet durch einen Gehalt an Polysiloxan-Ammonium-Derivaten der allgemeinen Formel I aus Anspruch 1 oder der allgemeinen Formel II aus Anspruch 2 in einer Menge von 0,1 bis 10 Gewichtsprozent, berechnet als reine Verbindung und bezogen auf das gesamte haarkosmetische Mittel und einen nichttoxischen Träger.

4. Haarwasch- und Haarbehandlungsmittel nach Anspruch 3, dadurch gekennzeichnet, daß sie neben dem Polysiloxanderivat andere übliche Wirkstoffe in den für den jeweiligen Verwendungszweck gebräuchlichen Mengen enthalten.

**Claims**

1. The use of polysiloxane-ammonium derivatives corresponding to the following general formula

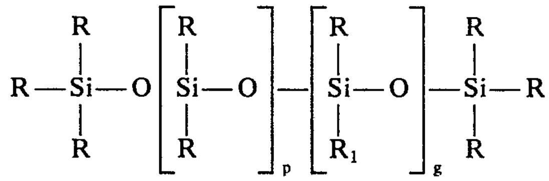

(I)

in which
R represents an alkyl radical containing from 1 to 4 C-atoms or an aryl radical, p is a number of from 0 to 300, g is a number of from 1 to 75 and $R_1$ represents the following radical

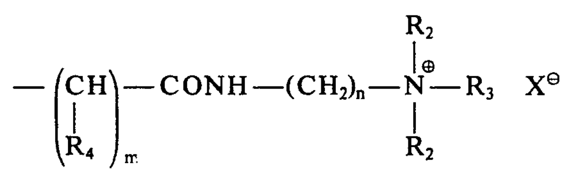

where $R_2$ is an alkyl or hydroxyalkyl radical containing from 1 to 3 carbon atoms, $R_3$ has the same meaning as $R_2$ or represents an aryl-$CH_2$ radical or the allyl radical, $R_4$ represents hydrogen or the methyl radical, $X^-$ represents the anions $Cl^-$, $Br^-$, $I^-$, $CH_3—SO_4^-$ or $C_2H_5—SO_4^-$, m is a number of from 2 to 10 and n a number of from 2 to 4, in hair-washing and hair-treatment preparations for improving the hairdressing properties of hair.

2. The use of polysiloxane-ammonium derivatives as claimed in Claim 1 corresponding to the following formula

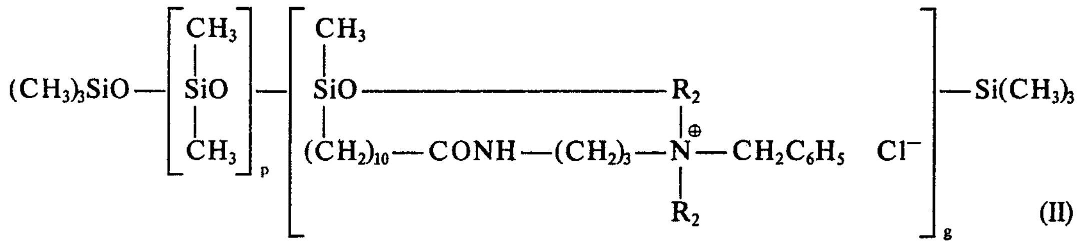

(II)

in which
$R_2$ represents the methyl or ethyl radical, p represents a number of from 10 to 50 and g a number of from 1 to 12

3. A hair-washing and hair-treatment preparation, characterised by a content of from 0.1 to 10% by weight, expressed as pure compound and based on the hair-cosmetic preparation as a whole and a non-toxic vehicle, of polysiloxane-ammonium derivatives corresponding to general formula I in Claim 1 or to general formula II in Claim 2.

4. A hair-washing and hair-treatment preparation as claimed in Claim 3, characterised in that, in addition to the polysiloxane derivative, it contains other standard active substances in the usual quantities for the particular application envisaged.

**Revendications**

1. Utilisation de dérivés d'ammonium de polysiloxanes de formule générale I

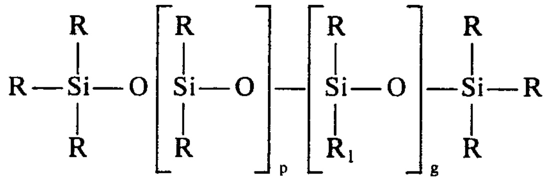

(I)

dans laquelle R représente un groupe alkyle en $C_1$—$C_4$ ou un groupe aryle, p est un nombre de 0 à 300, g un nombre de 1 à 75 et $R_1$ représente le reste

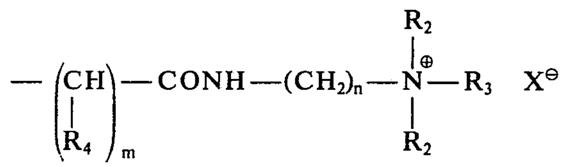

dans lequel $R_2$ représente un groupe alkyle ou hydroxyalkyle en $C_1$—$C_3$, $R_3$ représente un reste identique à $R_2$ ou un reste aryl-$CH_2$-ou le reste allyle, $R_4$ représente l'hydrogène ou le groupe méthyle, $X^-$ représente les anions $Cl^-$, $Br^-$, $I^-$, $CH_3SO_4^-$ ou $C_2H_5SO_4^-$, m est un nombre de 2 à 10 et n un nombre de 2 à 4,
dans des shampooings et produits pour le traitement de la chevelure en vue d'améliorer les propriétés des cheveux à l'égard des techniques de frisage.

2. Utilisation des dérivés d'ammonium de polysiloxanes selon la revendication 1 de formule II.

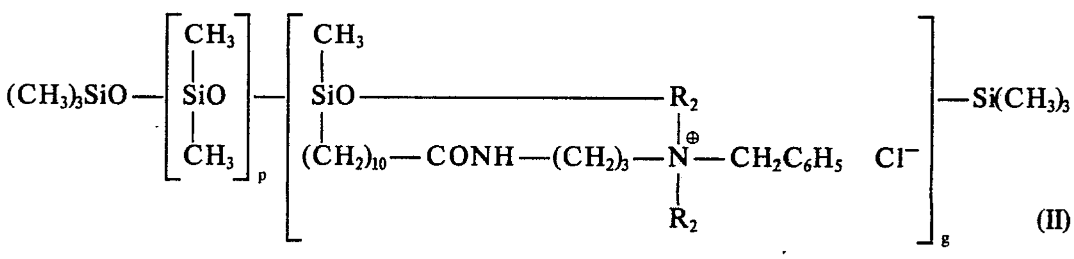

(II)

dans laquelle $R_2$ représente le groupe méthyle ou éthyle, p est un nombre de 10 à 50 et g un nombre de 1 à 12.

3. Shampooings et produits pour le traitement de la chevelure caractérisés en ce qu'ils contiennent des dérivés d'ammonium de polysiloxanes de formule générale I selon la revendication 1 ou de formule générale II selon la revendication 2 en quantité de 0,1 à 10% en poids, exprimé en composé pur et rapporté au produit cosmétique capillaire total, et un véhicule non toxique.

4. Shampooings et produits pour le traitement de la chevelure selon la revendication 3 caractérisés en ce qu'ils contiennent en plus du dérivé de polysiloxane d'autres substances actives usuelles aux quantités habituelles pour le but particulier recherché.